# EUROPÄISCHE PATENTANMELDUNG

(11) **EP 0 725 055 A1**
(43) Veröffentlichungstag der Anmeldung: **07.08.1996**
(21) Anmeldenummer: 96100876.0
(22) Anmeldetag: 23.01.1996
(51) Int. Cl.: C07C 69/738, C07C 67/333, C07C 67/08, C07C 67/347, C07C 67/00, C07C 27/02

(54) **Verfahren zur Herstellung von 5-Oxo-6-heptensäurealkylestern sowie neue Zwischenprodukte zu ihrer Herstellung**

(30) Priorität: 02.02.1995 DE 19503327
(71) Anmelder: BASF Aktiengesellschaft, 67063 Ludwigshafen (DE)
(72) Erfinder: Ebel, Klaus, Dr., D-68623 Lampertheim (DE); Eiermann, Matthias, Dr., D-67117 Limburgerhof (DE); Papkalla, Thomas, Dr., D-68165 Mannheim (DE)

(57) **Zusammenfassung**

Verfahren zur Herstellung von 5-Oxo-6-heptensäurealkylestern der allgemeinen Formel I 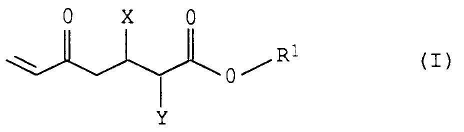
in der die Variablen X und Y für Wasserstoff oder eine Methylgruppe und der Rest R¹ für eine C₁-C₈-Alkylgruppe stehen, indem man
A) 5-Acetyl-2-norbornen mit einem Dialkylcarbonat CO(OR²)₂, wobei R² für eine C₁-C₄-Alkylgruppe steht, in Gegenwart einer Base zu einem 3-(2-Norbornen-5-yl)-3-oxopropionsäurealkylester der Formel II umsetzt, 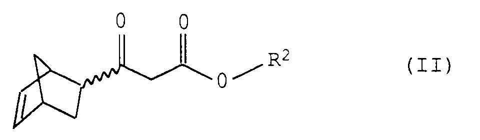
B) den Ester II in Gegenwart einer Base mit einer ungesättigten Verbindung der Formel III umsetzt, 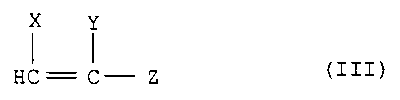 in der Z für eine CN- oder CO₂R³-Gruppe steht, wobei R³ für C₁-C₄-Alkyl steht, und zu einer 5-(2-Norbornen-5-yl)-5-oxopentansäure der Formel IV verseift, 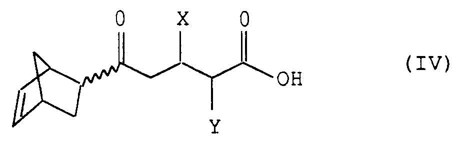
C) die Säure IV zu einem 5-(2-Norbornen-5-yl)-5-oxopentansäureester der Formel V verestert 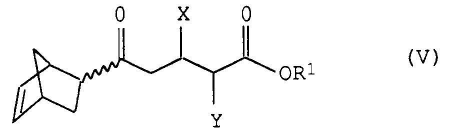
D) und diesen durch thermische Spaltung bei 300 bis 700°C in einen 5-Oxo-6-heptenalkylester I überführt.

Weiterhin betrifft die Erfindung neue Zwischenprodukte zur Herstellung der Ester I, deren Verwendung sowie ein verbessertes Verfahren zur Herstellung von 3-(2-Norbornen-5-yl)-3-oxopropionsäurealkylestern.

## Beschreibung

Die vorliegende Erfindung betrifft ein neues Verfahren zur Herstellung von 5-Oxo-6-heptensäurealkylestern der allgemeinen Formel I 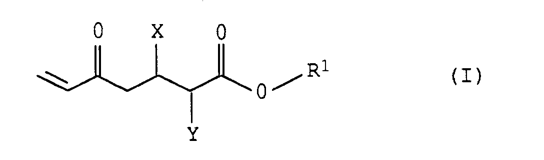
in der die Variablen X und Y für Wasserstoff oder eine Methylgruppe und der Rest R¹ für eine C₁-C₈-Alkylgruppe steht.

Weiterhin betrifft die Erfindung neue Zwischenprodukte zur Herstellung von Estern I, deren Verwendung sowie ein verbessertes Verfahren zur Herstellung von 3-(2-Norbornen-5-yl)-3-oxopropionsäurealkylestern.

5-Oxo-6-heptensäurealkylester I sind wertvolle Verbindungen für die Herstellung von Steroiden (GB-A 1 096 761, DE-A 20 16 750).

Der entsprechende Methylester ist durch Umsetzung von Glutarsäurechlorid, 2 Äquivalenten Aluminiumchlorid und Ethylen zu 7-Chlor-5-oxoheptansäuremethylester und anschließende Chlorwasserstoffabspaltung zugänglich (Houben-Weyl, Methoden der Organischen Chemie, Bd. 2/7a, S. 445). Glutarsäureverbindungen sind im industriellen Maßstab aber nur in geringen Mengen verfügbar.

Es bestand die Aufgabe, ein Herstellungsverfahren für 5-Oxo-6-heptensäurealkylester I zu finden, das den Einsatz von Glutarsäurederivaten vermeidet. Wegen der Polymerisationsneigung der Verfahrensprodukte I bestand weiterhin die Aufgabe, lagerstabile Verbindungen zu finden, aus denen die Ester I bei Bedarf freigesetzt werden können.

Demgemäß wurde ein Verfahren zur Herstellung der Ester I gefunden, das dadurch gekennzeichnet ist, daß man
A) 5-Acetyl-2-norbornen mit einem Dialkylcarbonat CO(OR²)₂, wobei R² für eine C₁-C₄-Alkylgruppe steht, in Gegenwart einer Base zu einem 3-(2-Norbornen-5-yl)-3-oxopropionsäurealkylester der Formel II umsetzt, 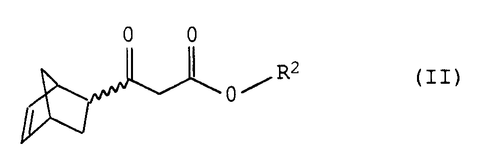
B) den Ester II in Gegenwart einer Base mit einer ungesättigten Verbindung der Formel III umsetzt, 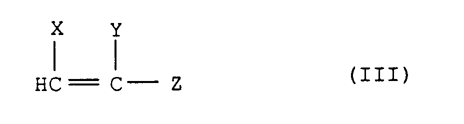 in der Z für eine CN- oder CO₂R³-Gruppe bedeutet, wobei R³ für C₁-C₄-Alkyl steht, und zu einer 5-(2-Norbornen-5-yl)-5-oxopentansäure der Formel IV verseift, 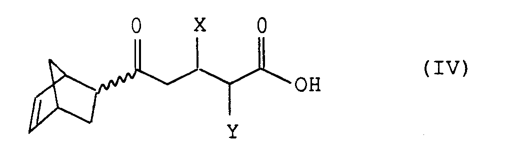
C) die Säure IV zu einem 5-(2-Norbornen-5-yl)-5-oxopentansäureester der Formel V verestert 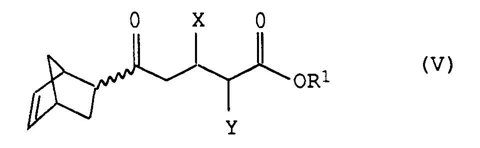
D) und diesen durch thermische Spaltung bei 300 bis 700°C in einen 5-Oxo-6-heptenalkylester I überführt.

Weiterhin wurden neue Verbindungen der Formeln IV und V gefunden, die lagerstabile Vorprodukte für die Verbindungen I sind.

Außerdem wurde ein verbessertes Verfahren zur Herstellung von 3-(2-Norbornen-5-yl)-3-oxopropionsäurealkylestern der Formel II gefunden, die selbst Zwischenprodukte im Verfahren zur Herstellung der Ester I sind.

### Verfahrensschritt A

5-Acetyl-2-norbornen kann als Gemisch der exo- und endo-Formen verwendet werden. Bevorzugt wird diese Verbindung aus Methylvinylketon und Cyclopentadien durch Diels-Alder-Reaktion hergestellt (z.B. Dinwiddie et al., J. Org. Chem. 30 (1965) 766). In Betracht kommt aber auch die Herstellung aus Dicyclopentadien (z.B. DE-A 16 18 145).

5-Acetyl-2-norbornen wird mit einem Di-(C₁-C₄-alkyl)carbonat, bevorzugt mit Dimethylcarbonat, und einer Base, z.B. einem Alkalimetallalkoholat wie Natriummethanolat, Natriumethanolat und Kaliummethanolat, einem Alkalimetallamid wie Natriumamid, vorzugsweise aber einem Alkalimetallhydrid, besonders Natriumhydrid, zu einem 3-(2-Norbornen-5-yl)-3-oxopropionsäurealkylester II umgesetzt.

Die Umsetzung ist an sich bekannt (vgl. Stork et al. Tetrahedron Lett. (1972) 2755), jedoch läßt sich die Reaktion beim Erhitzen aller Komponenten in einem inerten Lösungsmittel wegen der freigesetzten Wärmemenge schlecht kontrollieren und die Raumzeitausbeute läßt zu wünschen übrig. Diese Nachteile treffen auch auf eine Variante zu (Caselli et al., Austr. J. Chem. 35 (1982) 799), in der 5-Acetyl-2-norbornen zu einer Mischung aus der Base und dem Dialkylcarbonat gegeben wird.

Bevorzugt wird daher eine Ausführungsform, derzufolge 5-Acetyl-2-norbornen im Gemisch mit dem Dialkylcarbonat zur Base gegeben wird.

In der Regel wird die Base stöchiometrisch eingesetzt, d.h. üblicherweise 2 Äquivalente pro Äquivalent 5-Acetyl-2-norbornen, sie kann jedoch auch im Überschuß eingesetzt werden. Auch das Dialkylcarbonat wird im allgemeinen in einem Überschuß von 2 bis 6 Äquivalenten pro Äquivalent 5-Acetyl-2-norbornen verwendet.

Die Verwendung eines inerten Lösungsmittels, z.B. ein Ether wie 1,2-Dimethoxyethan, ist möglich, vorteilhafter ist aber, die Reaktion in einem Überschuß an Dialkylcarbonat durchzuführen, das gleichzeitig als Lösungsmittel dient.

Nach dem Zusammengeben der Komponenten wird die Mischung bei Temperaturen von beispielsweise 30 bis 70°C bis zur Vervollständigung der Rektion erhitzt. Typische Reaktionszeiten liegen bei 5 bis 20 h.

Die Isolierung des 3-Oxopropionsäureesters II erfolgt vorteilhaft durch Hydrolyse des Reaktionsgemisches, Extraktion des Produktes durch ein organisches Lösungsmittel wie Toluol und destillative Aufarbeitung.

### Verfahrensschritt B

Der Ester II - in der exo-Form, der endo-Form oder als Gemisch - kann an eine ungesättigte Verbindung III addiert werden. Im einzelnen handelt es sich dabei um Acrylnitril, Acrylsäuremethylester, Acrylsäureethylester, Acrylsäurepropylester, Acrylsäure-n-butylester, weiterhin um die entsprechenden Methacrylsäureester sowie um 2-Butencarbonsäureester.

Der Ester II und die ungesättigte Verbindung III werden in der Regel äquimolar verwendet.

Die Umsetzung wird in Gegenwart katalytischer Mengen einer Base, z.B. ein Alkalimetallalkoholat oder -hydroxid, bevorzugt Natriummethylat, vorgenommen. Die Reaktion kann in einem Lösungsmittel ausgeführt werden, bevorzugt ist aber eine Umsetzung in Substanz. Vorteilhaft wird die ungesättigte Verbindung III zu einem Gemisch aus dem Ester II und der Base gegeben.

Nach einer Reaktionszeit von beispielsweise 2 bis 20 h bei Temperaturen von im allgemeinen 20 bis 80°C wird das Reaktionsgemisch in situ zur 5-(2-Norbornen-5-yl)-5-oxopentansäure verseift.

Die Verseifung erfolgt in an sich bekannter Weise, beispielsweise durch Zugabe einer Base wie Natriumhydroxid zum Reaktionsgemisch und Erhitzen über mehrere Stunden.

Die Säuren IV können extraktiv isoliert werden.

### Verfahrensschritt C

Durch Veresterung nach an sich bekannten Methoden (s. Houben-Weyl, Methoden der Organischen Chemie, Band E5) kann die Säure IV in einen Ester V überführt werden, der C₁-C₈-Alkylreste trägt. Bevorzugt sind die Methyl- und Ethylester. Besonders vorteilhaft ist die Verwendung von katalytischen Mengen einer Säure, z.B. Mineralsäuren wie Schwefelsäure oder Sulfonsäuren wie p-Toluolsulfonsäure und C₁-C₈-Dialkylketalen wie 2,2-Dimethoxypropan oder einem C₁-C₈-Trialkylorthoester wie Orthoameisensäuremethylester.

Die Isolierung der Produkte V kann durch Neutralisation des Reaktionsgemisches mit einer wäßrigen Base, Extraktion mit einem organischen Lösungsmittel und Destillation erfolgen. Die Ester V sind bei Raumtemperatur lagerstabile Verbindungen, die auch nach Monaten keine merklichen Anzeichen einer unerwünschten Polymerisation zeigen.

### Verfahrensschritt D

Die Ester V können thermisch in Cyclopentadien und die Verfahrensprodukte I gespalten werden. Für diese Retro-Diels-Alder-Reaktion sind Temperaturen von 300 bis 700°C erforderlich, bevorzugt sind 450 bis 600°C. Die Reaktion erfolgt in der Gasphase. Der Druck beträgt während dieses Verfahrensschritts im allgemeinen 0,01 bis 10 mbar, bevorzugt 1 bis 2 mbar. Das dabei anfallende Rohprodukt wird bevorzugt destillativ auf die Verfahrensprodukte I aufgearbeitet.

Das erfindungsgemäße Verfahren zur Herstellung von 5-Oxo-6-heptensäurealkylestern I kann ohne die Verwendung von Glutarsäurederivaten ausgeführt werden. Es führt in guten Ausbeuten zu den gewünschten Verfahrensprodukten. Die dabei gebildeten Zwischenprodukte, die 5-(2-Norbornen-5-yl)-5-oxopentansäuren IV sowie insbesondere die entsprechenden Alkylester V stellen bequem zu handhabende, bei Raumtemperaturen stabile Lagerformen der Ester I dar.

### Beispiele

### Verfahrensschritt A

### Beispiel 1: Herstellung von exo/endo-3-(2-Norbornen-5-yl)-3-oxopropionsäuremethylester

66,7 g 60 %iges Natriumhydrid in Weißöl wurden in 200 ml wasserfreiem 1,2-Dimethoyethan unter Stickstoff bei Rückflußtemperatur tropfenweise mit einer Mischung von 644 g Dimethylcarbonat und 108,8 g exo/endo-5-Acetyl-2-norbornen versetzt und anschließend 16 bis 20 h zum Sieden erhitzt. Nach Abkühlen wurde das Gemisch auf Eis/Natriumhydrogencarbonat gegossen, mit 10 %iger Salzsäure neutralisiert und zweimal mit je 200 ml Toluol extrahiert. Die gesammelten organischen Phasen wurden mit Wasser gewaschen, mit Magnesiumsulfat getrocknet und im Vakuum destilliert.
Sdp. 85 bis 90°C/0,5 mbar, Ausbeute 113,9 g (73 %).

### Beispiel 2: Herstellung von exo/endo-3-(2-Norbornen-5-yl)-3-oxopropionsäureethylester

160 g Natriumethylat wurden in 350 ml wasserfreiem 1,2-Dimethoxyethan unter Stickstoff bei Rückflußtemperatur tropfenweise mit einer Mischung von 1,06 kg Diethylcarbonat und 136 g exo/endo-5-Acetyl-2-norbornen versetzt und anschließend 16 bis 20 h zum Sieden erhitzt. Die Aufarbeitung erfolgte wie in Beispiel 1.
Sdp. 94 bis 111°C/0,5 mbar, Ausbeute 75 g (36 %).

### Verfahrensschritt B

### Beispiel 3: Herstellung von exo/endo-5-(2-Norbornen-5-yl)-5-oxopentansäure

Zu 2,0 g Natriummethylat und 130,4 g exo/endo-3-(2-Norbornen-5-yl)-3-oxopropionsäuremethylester wurden unter Stickstoff bei 40°C 58,0 g Acrylsäuremethylester gegeben. Nach 16 h bei 40°C wurden 550 ml 5 N wäßrige Natronlauge zugesetzt und 2 h auf Siedetemperatur erhitzt. Nach Abkühlen wurde zweimal mit je 200 ml Ether extrahiert. Aus den Etherphasen konnten geringe Mengen an exo/endo-5-Acetyl-2-norbornen gewonnen werden. Die wäßrige Phase wurde mit konzentrierter Salzsäure auf pH=1 gestellt und dreimal mit je 100 ml Ether extrahiert. Die vereinigten organischen Extrakte wurden mit 100 ml 5 %iger wäßriger Salzsäure gewaschen, mit Magnesiumsulfat getrocknet und eingeengt. Rohaustrag (exo/endo ≈ 1:1) 128,0 g.
¹H-NMR(CDCl₃): 1,2-1,6 (m, 6H); 1,7-2,0 (m, 6H); 2,3-2,7 (m, 9H); 2,95 (br.s, 2H); 2,98 (br.s, 1H); 3,00 (m, 1H); 3,20 (br.s, 1H); 5,80 (m, 1H, -CH=); 6,18 (m, 3H, -CH=); 8,6 (br., -COOH).
¹³C-NMR (CDCl₃): 18,61; 18,75; 27,46; 29,22; 33,07; 33,11; 40,41; 41,28; 41,68; 42,67; 45,53; 45,90; 46,03; 49,96; 50,80; 51,65; 131,16; 135,83; 137,92; 138,40; 179,33; 179,36; 210,48; 212,26.

### Beispiel 4: Herstellung von exo/endo-5-(2-Norbornen-5-yl)-5-oxopentansäure

Die Umsetzung erfolgte analog zu Beispiel 3 unter Ersatz des Acrylsäuremethylesters durch 35,7 g Acrylnitril. Die Reaktionsdauer mit der 5 N wäßrigen Natronlauge verlängerte sich auf 48 h.

### Verfahrensschritt C

### Beispiel 5: Herstellung von exo/endo-5-(2-Norbornen-5-yl)-5-oxopentansäuremethylester

128 g des Rohaustrags nach Beispiel 3 wurden mit 73,8 g 2,2-Dimethoxypropan und 12,3 g p-Toluolsulfonsäure in 290 ml Methanol 3 h zum Sieden erhitzt. Nach Abkühlen wurde eingeengt, mit 2 N wäßriger Natronlauge alkalisch gestellt und zweimal m it je 200 ml Ether extrahiert. Die vereinigten Etherphasen wurden mit 100 ml Wasser gewaschen, mit Magnesiumsulfat getrocknet und destilliert. Destillat (exo/endo ≈ 1:1, Sdp. 115 bis 120°C/1 mbar) 69 g (46 % Ausbeute über Beispiel 3 und 5).
¹H-NMR (CDCl₃): 1,2-1,6 (m, 6H); 1,7-2,0 (m, 6H); 2,3-2,4 (m, 5H); 2,5-2,7 (m, 4H); 2,90 (br.s, 2H); 2,95 (br.s, 1H); 3,00 (m, 1H); 3,25 (br.s, 1H); 3,65 (s, 6H, -OCH₃); 5,80 (m, 1H, -CH=); 6,15 (m, 3H, -CH=).
¹³C-NMR (CDCl₃): 18,94; 19,07; 27,37; 29,16; 33,00; 33,04; 40,42; 41,32; 41,70; 42,69; 45,63; 45,85; 45,98; 49,94; 50,67; 51,41; 51,54; 131,28; 135,87; 137,69; 138,17; 173,48; 209,65; 211,54.
IR (KBr): 1702; 1735 cm⁻¹.

### Beispiel 6: Herstellung von exo/endo-5-(2-Norbornen-5-yl)-5-oxopentansäuremethylester

8,5 g des Rohaustrags nach Beispiel 3 wurden mit 2 ml Methanol, 0,2 ml konzentrierter Salzsäure und 0,9 g wasserfreiem Calciumchlorid 30 Minuten zum Sieden erhitzt. Nach Trennung der Phasen wurde die wäßrige Phase nach Zusatz von Natriumhydrogencarbonat mit 10 ml Toluol extrahiert. Die vereinigten organischen Phasen wurden mit Magnesiumsulfat getrocknet und destilliert. Destillat (exo/endo ≈ 1:1) 4,5 g (46 % Ausbeute über Beispiel 3 und 6).

### Verfahrensschritt D

### Beispiel 7: Herstellung von 5-Oxo-6-heptensäuremethylester

29,9 g exo/endo-5-(2-Norbornen-5-yl)-5-oxopentansäuremethylester wurden bei 538 bis 548°C während 3 h bei 1 mbar Druck in einen mit Quarzglasringen (10 mm Länge x 4 mm Durchmesser) gefüllten, vertikalen Quarzglas-Rohrreaktor (30 cm Länge x 3 cm Durchmesser) von oben zugegeben. Das Produkt wurde am unteren Ende des Reaktors abgenommen, kondensiert (Rohaustrag 19,2 g) und anschließend bei 1 mbar destilliert. Destillat 15,8 g (75 %).

## Patentansprüche

1. Verfahren zur Herstellung von 5-Oxo-6-heptensäurealkylestern der allgemeinen Formel I 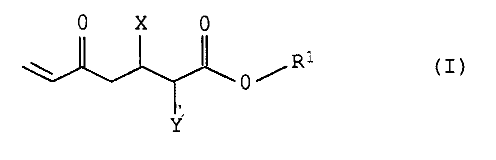 in der die Variablen X und Y für Wasserstoff oder eine Methylgruppe und der Rest R¹ für eine C₁-C₈-Alkylgruppe stehen, dadurch gekennzeichnet, daß man
A) 5-Acetyl-2-norbornen mit einem Dialkylcarbonat CO(OR²)₂, wobei R² für eine C₁-C₄-Alkylgruppe steht, in Gegenwart einer Base zu einem 3-(2-Norbornen-5-yl)-3-oxopropionsäurealkylester der Formel II umsetzt, 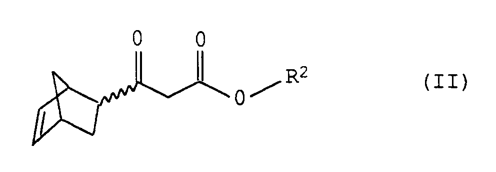
B) den Ester II in Gegenwart einer Base mit einer ungesättigten Verbindung der Formel III umsetzt, 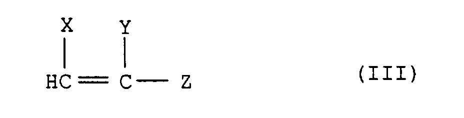 in der Z für eine CN- oder CO₂R³-Gruppe steht, wobei R³ für C₁-C₄-Alkyl steht, und zu einer 5-(2-Norbornen-5-yl)-5-oxopentansäure der Formel IV verseift, 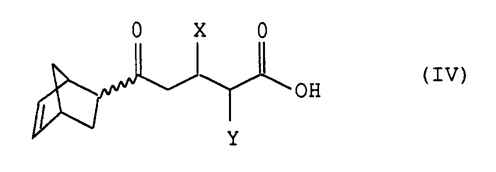
C) die Säure IV zu einem 5-(2-Norbornen-5-yl)-5-oxopentansäureester der Formel V verestert 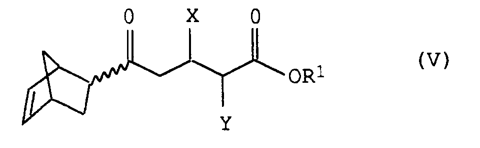
D) und diesen durch thermische Spaltung bei 300 bis 700°C in einen 5-Oxo-6-heptenalkylester I überführt.

2. Verfahren nach Anspruch 1, dadurch gekennzeichnet, daß man 5-Acetyl-2-norbornen aus Methylvinylketon und Cyclopentadien herstellt.

3. Verfahren nach Anspruch 1 oder 2, dadurch gekennzeichnet, daß man 5-Oxo-6-heptensäuremethylester herstellt.

4. Verfahren nach den Ansprüchen 1 bis 3, dadurch gekennzeichnet, daß man die Veresterung in Verfahrensschritt C) in Gegenwart katalytischer Mengen einer Säure mit einem C₁-C₈-Dialkylketal oder einem C₁-C₈-Trialkylorthoester vornimmt.

5. 1,4-Ketocarbonsäurederivate der Formeln IV und V 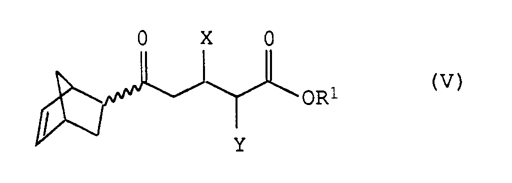 in denen X und Y für Wasserstoff oder eine Methylgruppe und R¹ für eine C₁-C₈-Alkylgruppe stehen.

6. Verwendung von 5-(2-Norbornen-5-yl)-5-oxopentansäureestern der Formel V als lagerstabile Vorstufe für 5-Oxo-6-heptensäurealkylester der Formel I.

7. Verfahren zur Herstellung von 3-(2-Norbornen-5-yl)-3-oxopropionsäurealkylestern der Formel II durch Umsetzung von 5-Acetyl-2-norbornen mit einem Dialkylcarbonat und einer Base, dadurch gekennzeichnet, daß man 5-Acetyl-2-norbornen im Gemisch mit dem Dialkylcarbonat zur Base gibt.

8. Verfahren nach den Ansprüchen 1 bis 4, dadurch gekennzeichnet, daß man in der Reaktionsstufe A) entsprechend dem Verfahren gemäß Anspruch 7 verfährt.
